# EUROPÄISCHE PATENTSCHRIFT

(11) **EP 1 504 103 B1**
(45) Veröffentlichungstag und Bekanntmachung des Hinweises auf die Patenterteilung: **23.11.2011**
(21) Anmeldenummer: 03729992.2
(22) Anmeldetag: 08.05.2003
(51) Int. Cl.: C12N 15/81, C12N 1/19

(54) **PROMOTOREN MIT VERÄNDERTER TRANSKRIPTIONSEFFIZIENZ AUS DER METHYLOTROPHEN HEFE HANSENULA POLYMORPHA**
PROMOTERS HAVING A MODIFIED TRANSCRIPTION EFFICIENCY AND DERIVED FROM METHYLOTROPHIC YEAST
PROMOTEURS AYANT UNE EFFICACITÉ DE TRANSCRIPTION MODIFIÉE, DERIVÉS DE LEVURE METHYLOTROPHE HANSELUNA POLYMORPHA

(30) Priorität: 10.05.2002 DE 10220894
(43) Veröffentlichungstag der Anmeldung: 09.02.2005
(73) Patentinhaber: RHEIN BIOTECH GESELLSCHAFT FÜR NEUE BIOTECHNOLOGISCHE PROZESSE UND PRODUKTE MBH, 40595 Düsseldorf (DE)
(72) Erfinder: SUCKOW, Manfred, 40593 Düsseldorf (DE)
(74) Vertreter: Herzog, Martin
(86) Internationale Anmeldenummer: PCT/EP2003/004844
(87) Internationale Veröffentlichungsnummer: WO 2003/095653

(56) Entgegenhaltungen:
- GOEDECKE S ET AL: "IDENTIFICATION OF SEQUENCES RESPONSIBLE FOR TRANSCRIPTIONAL REGULATION OF THE STRONGLY EXPRESSED METHANOL OXIDASE-ENCODING GENE IN HANSENULA POLYMORPHA" GENE, ELSEVIER BIOMEDICAL PRESS. AMSTERDAM, NL, Bd. 139, Nr. 1, 1994, Seiten 35-42, XP002226643 ISSN: 0378-1119
- PEREIRA GONCALO G ET AL: "Conserved regulation of the Hansenula polymorpha MOX promoter in Saccharomyces cerevisiae reveals insights in the transcriptional activation by Adr1p." EUROPEAN JOURNAL OF BIOCHEMISTRY, Bd. 238, Nr. 1, 1996, Seiten 181-191, XP001154636 ISSN: 0014-2956
- SUCKOW M ET AL: "The Activation Specifities of Wild-type and Mutant Gcn4p in vivo can be Different from the DNA Binding Specificities of the Corresponding bZip Peptides in vitro" JOURNAL OF MOLECULAR BIOLOGY, LONDON, GB, Bd. 276, 1998, Seiten 887-902, XP002189244 ISSN: 0022-2836 in der Anmeldung erwähnt
- AMUEL, CARSTEN ET AL: "Analysis of heat shock promoters in Hansenula polymorpha: the TPS1 promoter, a novel element for heterologous gene expression" BIOTECHNOLOGY AND BIOPROCESS ENGINEERING (2000), 5(4), 247-252 , XP001154638
- PHONGDARA A ET AL: "Cloning and characterization of the gene encoding a repressible acid phosphatase (PHO1) from the methylotrophic yeast Hansenula polymorpha." APPLIED MICROBIOLOGY AND BIOTECHNOLOGY, Bd. 50, Nr. 1, Juli 1998 (1998-07), Seiten 77-84, XP002253409 ISSN: 0175-7598
- VAN DIJK RALF ET AL: "The methylotrophic yeast Hansenula polymorpha: A versatile cell factory." ENZYME AND MICROBIAL TECHNOLOGY, Bd. 26, Nr. 9-10, Juni 2000 (2000-06), Seiten 793-800, XP000994959 ISSN: 0141-0229
- COX H ET AL: "Constitutive expression of recombinant proteins in the methylotrophic yeast Hansenula polymorpha using the PMAI promoter" YEAST, CHICHESTER, SUSSEX, GB, Bd. 16, Nr. 13, 30. September 2000 (2000-09-30), Seiten 1191-1203, XP002226641 ISSN: 0749-503X in der Anmeldung erwähnt
- GELLISSEN GERD ET AL: "Application of yeasts in gene expression studies: A comparison of Saccharomyces cerevisiae, Hansenula polymorpha and Kluyveromyces lactis: A review" GENE: AN INTERNATIONAL JOURNAL ON GENES AND GENOMES, ELSEVIER SCIENCE PUBLISHERS, BARKING, GB, Bd. 190, Nr. 1, 1997, Seiten 87-97, XP002164545 ISSN: 0378-1119

## Beschreibung

Die vorliegende Erfindung betrifft eine Desoxyribonukleinsäure (DNA), umfassend mindestens eine Promotorsequenz, die von einem Wildtyp-Promotor einer methylotrophen Hefe abgeleitet ist, deren Transkriptionseffizienz im Vergleich zu der Effizienz des Wildtyp-Promotors moduliert ist Ferner betrifft die vorliegende Erfindung Wirtszellen, Expressionsvektoren, Kits und Verfahren zum Herstellen von Proteinen unter Verwendung der erfindungsgemäßen DNA sowie verschiedene Verwendungen derselben und ein Verfahren zur Herstellung von Expressionsvektoren.

Pilze und insbesondere Hefen stellen aufgrund ihrer Fähigkeit zur säugetierähnlichen co- und posttranslationalen Modifikation von Proteinen attraktive Systeme für die Produktion rekombinanter Proteine dar. Zur Herstellung rekombinanter Proteine wird häufig die kodierende Sequenz eines Gens für ein Protein von Interesse unter der Kontrolle eines geeigneten heterologen Promotors exprimiert. Als besonders vorteilhaft haben sich dabei die induzierbaren Promotoren aus methylotrophen Hefen erwiesen.

Methylotrophe Hefen umfassen beispielsweise die Gattungen *Candida*, *Hansenula*, *Pichia und Torulopsis*. Ihre Promotoren zeichnen sich durch eine außergewöhnlich starke Transkriptionsinduktion sowie gute und einfache Regulierbarkeit aus. Besonders vorteilhafte Promotoren sind die in vivo für die Regulation des Methanol-Metabolismus verantwortlichen Promotoren. Methylotrophe Hefen katalysieren die Oxidation von Methanol über die Zwischenstufen des Formaldehyds und Formiats zu Kohlendioxid, wobei diese Reaktionen jeweils durch eine Alkohol- oder Methanoloxidase (Aoxp bzw. Moxp), eine Formaldehyddehydrogenase (Fmdp) und eine Formiatdehydrogenase (Fmdhp) katalysiert werden. Das im ersten Schritt entstehende Wasserstoffperoxid wird durch Katalase abgebaut Die C1-Verbindung wird durch die Transketolasereaktion von Xylulose-5(P) mit Formaldehyd assimiliert. Diese Reaktion wird durch die Dihydroxyaceton-Synthase (Dhasp) katalysiert. Die unter der Kontrolle der *AOX-* bzw. *MOX-, FMD*- und *DHAS*-Promotoren exprimierten Enzyme Aoxp bzw. Moxp, Fmdhp und Dhasp stellen in methanolinduzierten Zellen bis zu 40% des gesamten Zellproteins dar. Diese Promotoren werden daher in Fachkreisen auch als Superpromotoren bezeichnet. Die meisten der Promotoren für die genannten Gene sind durch Glukose reprimierbar und daher sehr gut individuell regulierbar.

Ein weiterer sehr starker Promotor aus methylotrophen Hefen ist der TPS1-Promotor, der die Expression des Trehalose-6-Phosphat-Synthase-Gens steuert und hitzeinduzierbar ist. Tps1p katalysiert die Umsetzung von Glukose-6-Phosphat (GLU6P) und UDP-Glukose (UDPG) zu Trehalose-6-Phosphat und UDP. Behandelte Hefezellen akkumulleren während eines einstündigen Hitzeschocks von 27°C auf 40°C Trehalose und bauen dadurch eine erhöhte Thermotoleranz auf.

Methylotrophe Hefen haben darüber hinaus außergewöhnlich starke konstitutive Promotoren. Zwei dieser sehr starken konstitutiven Promotoren sind GAPDH (Glycerinaldehydphosphat-dehydrogenase) und *PMA1* (Plasmamembrane-gebundene H⁺-ATPase 1).

Bei der rekombinanten Darstellung von heterologen Proteinen bestimmt sowohl die Aktivität der Promotorsequenz, deren Regulation wie auch deren Integration in das Genom einer , geeigneten Wirtszelle, die Wirtschaftlichkeit und Effizienz des Verfahrens. Im Allgemeinen ist eine besonders hohe Transkriptionsaktivität wünschenswert, In manchen Fällen kann eine zu hohe Transkriptionsaktivität jedoch dazu führen, dass die Wirtszelle geschädigt wird oder abstirbt, bevor alle heterologen Genprodukte der zur Aktivität notwendigen co- und post-translationalen Modifikation zugeführt werden konnten. Insbesondere toxische Proteine müssen in ihrer Menge dahingehend reguliert werden, dass die toxische Wirkung bis zur optimalen Ausbeute transkribierter und posttranslational modifizierter Proteine minimiert wird. Eine solche Regulation ist auch mit den bekannten durch Methanol induzierbaren Promotoren schwierig zu bewerkstelligen.

Aufgabe der vorliegenden Erfindung ist es daher, eine eine Promotorsequenz umfassende DNA zur Verfügung zu stellen, die den individuellen Anforderungen methylotropher Hefetranskriptionssysteme gerecht wird.

Erfindungsgemäß wird diese Aufgabe durch eine DNA gelöst, wie in Anspruch 1 definiert.

Es wurde festgestellt, dass die MOX-, FMD- und TPS1-Promotoren von *Hansenula polymorpha* in ihrer Transkriptionseffizienz moduliert werden können. Eine solche Modulation der Transkriptionseffizienz kann sowohl durch die Verstärkung als auch durch Verminderung der Aktivität der Promotorsequenz und/oder eine vermehrte oder verminderte Integration der Promotorsequenz in das Genom einer geeigneten Wirtszelle zustande kommen.

Unter einem Wildtyp-Promotor aus einer methylotrophen Hefe ist ein solcher Promotor zu verstehen, der die gleiche DNA-Sequenz und die gleiche Transkriptionseffizienz aufweist wie die Promotoren aus einer natürlich vorkommenden methylotrophen Hefe. Dem Fachmann sind geeignete Wildtyp-Promotoren aus der Fachliteratur, durch öffentliche Hinterlegung und durch Veröffentlichung in Datenbanken bekannt. So sind beispielsweise die Promotoren *FMD*, *MOX* und *TPS1* in den europäischen Patentanmeldungen EP299108, EP173378 und EP1151112 offenbart;

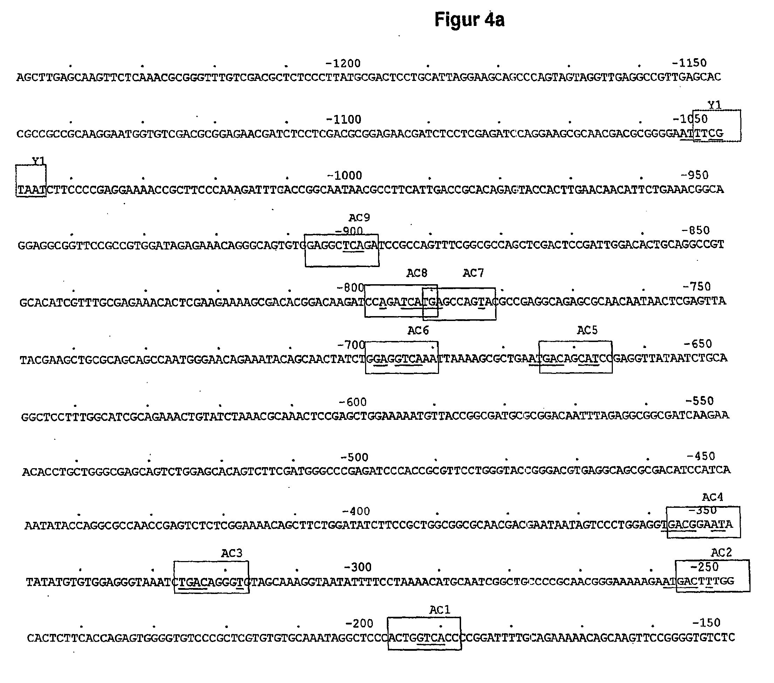

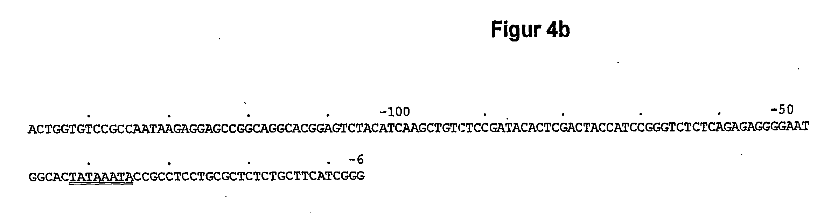

Beispiele für Sequenzen bestimmter Wildtyp-*FMD*-, *MOX-* und *TPS1*-Promotoren können den Figuren 4 bis 6 entnommen werden. Da die Sequenzanalyse insbesondere vor zehn : und mehr als zwanzig Jahren manchmal fehlerbehaftet war, aber mögliche Fehler durch Überprüfung mittels standardisierter Routinemethoden ohne weiteres erkennbar sind, werden unter Wildtyp-Promotoren im Sinne der Erfindung nicht nur die im Stand der Technik durch Beschreibung offenbarten, sondern auch die tatsächlich zugänglichen Wildtyp-Promotoren verstanden.

Unter Transkriptionseffizienz ist die Produktion von Transkript, d. h. mRNA pro Zeiteinheit zu verstehen. Für den Zweck dieser Anmeldung wird sie als die Menge eines heterologen Proteins bestimmt, das in einer geeigneten Wirtszelle unter Kontrolle eines Promotors pro Zeiteinheit exprimiert wird, wobei angenommen wird, dass die Translationseffizienz nur von der Menge verfügbaren Transkriptes abhängt. Die Transkriptionseffizienz wird beispielsweise durch eine Mengenbestimmung des heterologen Proteins oder durch Auswertung der Signale eines Reportergens wie *lacZ* oder Phytase zu verschiedenen Zeitpunkten durch Standardverfahren bestimmt.

Besonders bevorzugt ist eine erfindungsgemäße DNA, bei der die Transkriptionseffizienz der Promotorsequenz im Vergleich zu der Effizienz des Wildtyp-Promotors durch Einfügen einer DNA-Bindungsstelle erhöht ist. In einer Ausführungsform weisen die erfindungsgemäßen DNA-Sequenzen gegenüber den Wildtyp-Promotoren sogar eine erheblich erhöhte Transkriptionseffizienz auf. Dies ist um so überraschender, als die Wildtyp-Promotoren methylotropher Hefen bereits zu den effizientesten Promotoren überhaupt zählen. Eine weitere und vor allem so deutliche Steigerung ist deshalb höchst erstaunlich.

In einer bevorzugten Ausführungsform betrifft die Erfindung eine DNA, deren Promotorsequenz im Vergleich zum Wildtyp-Promotor eine um mindestens den Faktor 1,5, vorzugswelse um mindestens den Faktor 2, besonders bevorzugt um mindestens den Faktor 2,5 erhöhte Transkriptionseffizienz aufweist.

In einer anderen bevorzugten Ausführungsform betrifft die Erfindung eine DNA, deren Promotorsequenz im Vergleich zum Wildtyp-Promotor eine verminderte Transkriptionseffizienz aufweist ist vorzugsweise eine um mindestens den Faktor 0,5 verminderte Transkriptionseffizienz.

Die Promotorsequenzen einer efindungsgemäßen DNA sind abgeleitet von einem Promotor aus der Gattung *Hansenula polymorpha.*

Die Promotorsequenz einer erfindungsgemäßen DNA ist aus Promotoren der Gruppe der *MOX-, FMD-,* und *TPS1-,* abgeleitet.

Vorzugsweise umfasst eine erfindungsgemäße DNA mindestens eine Promotorsequenz, die durch die Insertion, Deletion und/oder den Austausch mindestens einer Base, vorzugsweise durch den Austausch von ein oder zwei Basen, von einem Wildtyp-Promotor abgeleitet ist.

Es wurde überraschenderweise festgestellt, dass die Wildtyp-Promotorsequenzen methylotropher Hefen DNA-Bereiche aufweisen, die den DNA-Bindungsstellen für Transkriptionsfaktoren der Klasse der bZip-Proteine ähnlich, aber nicht identisch sind, wobei diese DNA-Bereiche in der methylotrophen Hefe natürlicherweise keine DNA-Bindungsfunktion haben. Diese sog. kryptischen, d. h. ähnlichen aber nicht funktionsfähigen, DNA-Bindungsstellen für Transkriptionsfaktoren der Klasse der bZip-Proteine stellen eine bevorzugte Möglichkeit zur Veränderung der Promotortranskriptionseffizienz dar. Es wurde festgestellt, dass eine Modifikation dieser kryptischen DNA-Bindungsstellen durch Deletion, Insertion oder Austausch einzelner oder mehrerer Basenpaare die Promotortranskriptionseffizienz methylotropher Hefen erheblich verändern kann. Dabei zeigt insbesondere die Ausgestaltung dieser DNA-Bindungsstelle als palindrome DNA eine starke Veränderung der Transkriptionsaktivität.

In einer besonders bevorzugten Ausführungsform umfasst die vorliegende Erfindung daher eine DNA, deren Promotorsequenz mindestens eine DNA-Bindungsstelle, vorzugsweise eine palindrome DNA-Bindungsstelle für Transkriptionsfaktoren der Klasse der bZip-Proteine aufweist.

Transkriptionsfaktoren der Klasse der bZip-Proteine werden beispielsweise in Suckow et al., J. Mol. Biol. (1998) 276, 887-902 beschrieben. BZip-Proteine weisen eine basische Region auf, die für die DNA-Erkennung verantwortlich ist, sowie einen C-terminalen Leucin-"Reißverschluss", der für die spezifische Dimerbildung dieser Faktoren verantwortlich ist. Korrespondierende DNA-Bindungsstellen für die bZip-Proteine sind meist palindrome oder pseudopalindrome Sequenzen mit 10 oder 9 Basenpaaren.

Des weiteren wurde festgestellt, dass die Transkriptionseffizienz von Promotorsequenzen methylotropher Hefen durch Veränderung von DNA-Bereichen moduliert werden kann, deren Motiv den palindromen DNA-Bindungsstellen von Zinkfingerproteinen ähnelt. Diese DNA-Bereiche können durch Deletion, Insertion oder Austausch mindestens eines Basenpaares abgeändert werden, wobei vorzugsweise eine palindrome DNA-Bindungsstelle für Zinkfingerproteine entsteht. In einer weiteren bevorzugten Ausführungsform umfasst die Promotorsequenz einer erfindungsgemäßen DNA daher mindestens eine DNA-Bindungsstelle, vorzugsweise eine palindrome DNA-Bindungsstelle, für Zinkfingerproteine.

Sowohl die DNA-Motive für Transkriptionsfaktoren der Klasse der bZip-Proteine wie auch für Zinkfingerproteine sind dem Fachmann auf dem Gebiet der Molekularbiologie geläufig. Der Fachmann kann somit In Ausführung der vorliegenden Erfindung die Sequenzen von Wildtyp-Promotoren auf DNA-Sequenzen hin absuchen, die den genannten Motiven nahe kommen und durch Standardverfahren in funktionelle und/oder identische Bindungsstellen für bZip-Proteine oder Zinkfingerproteine abändern.

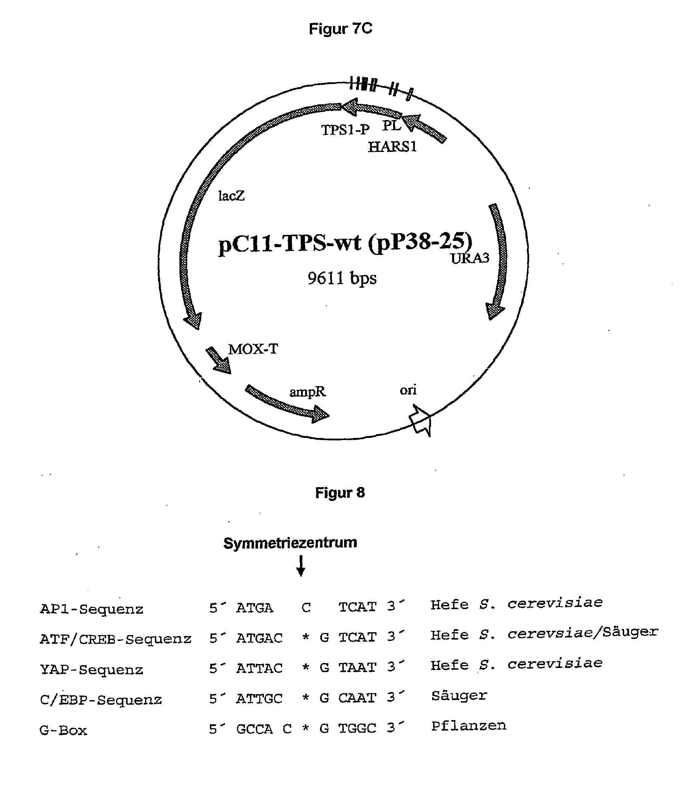

Besonders bevorzugt sind erfindungsgemäße DNA-Sequenzen, deren Promotorsequenz mindestens eine DNA-Bindungsstelle aufweist, die im wesentlichen der ATF/CREB-Bindungsstelle, der YAP-Bindungsstelle, der C/EBP-Bindungsstelle, der AP1-Bindungsstelle oder der G-Box entspricht Beispielhafte Ausführungsformen dieser bevorzugten Bindungsstellen werden In Figur 8 gezeigt (Beispiel für AP1 oder G-Box) "Im wesentlichen" bedeutet dabei, dass pro Strang nicht mehr als drei, bevorzugt zwei Basen oder nur eine Base von der Sequenz an der genannten Bindungsstelle abweich(t)en.

Die Promotorsequenz einer erfindungsgemäßen DNA enthält mindestens eine palindromisierte DNA-Sequenz, die von den in SEQ ID NO: 1-4, 11, 13, 16 oder 20 angegebenen Sequenzen abgeleitet ist.

**Tabelle 1**

| **SEQ ID NO:** | **Sequenz** | **Referenz-Bezeichnung** |
|---|---|---|
| 1 | CACTGGTCAC | *FMD* AC1 |
| 2 | ATGACTTTGG | *FMD* AC2 |
| 3 | CTGACAGGGT | *FMD* AC3 |
| 4 | GTGACGGAAT | *FMD* AC4 |
| 11 | ATGGTGTCAT | *MOX* AC1 |
| 13 | CGAATGTAAT | *MOX* Y1 |
| 16 | ATGGTGAGAT | *TPSI* AC1 |
| 20 | ATTGACCAAT | *TPSI* C1 |

Tabelle 1 zeigt die bevorzugten kryptischen bZip-Sequenzen in den Promotoren *FMD*, *MOX* und *TPS1* gemäß den Figuren 4 bis 6.

Vorzugsweise umfasst die palindromisierte Sequenz mindestens 8 bis 20, vorzugsweise 8 bis 12, besonders bevorzugt 10 Basen.

Die palindromisierte Sequenz kann symmetrisch oder unsymmetrisch sein. Vorzugsweise ist sie symmetrisch. Die palindromisierte Sequenz weicht nicht mehr als maximal in 2 Basen pro Strang, besonders bevorzugt in nur einer Base und ganz besonders bevorzugt gar nicht von einer vollständig palindromen Sequenz ab.

Die erfindungsgemäßen DNA-Moleküle können beispielsweise nach gängigen Verfahren synthetisch hergestellt, oder aber aus geeigneten DNA-Bibliotheken isoliert und anschliessend mutiert werden. Die Herstellung solcher Bibliotheken ist dem Fachmann ebenfalls bekannt. Eine Isolierung erfolgt zum Beispiel, indem eine Sonde mit einer Länge von mindestens 200 - 400 bp und der Sequenz eines Wildtyp-Promotors einer methylotrophen Hefe hergestellt und damit eine DNA-Bibliothek, insbesondere eine genomische DNA-Bibliothek gescreent wird. Eine solche Sonde kann unter Verwendung von geeigneten Primern, welche jeweils vorzugsweise mindestens 20 - 21 bp lang sind und geeignete Sequenzen aufweisen, und genomischer oder cDNA von methylotrophen Hefen als "Template", mittels PCR (Polymerase Chain Reaction) hergestellt werden.

Sonden können beispielsweise synthetisiert oder durch Fragmentierung vorhandener Wildtyp-Promotor DNA hergestellt werden. Es ist auch möglich, direkt mit Sonden entsprechenden Teilen der Promotorsequenz zu screenen. Ein solches Vorgehen ist aber infolge mangelhafter Konservierung der Sequenz innerhalb nicht kodierender Teile weniger bevorzugt.

Die erfindungsgemäßen DNA-Moleküle können weiterhin mindestens eine unter der Transkriptionskontrolle der Promotorsequenz stehende DNA-Sequenz für ein homologes und/oder heterologes Gen umfassen.

Unter einem "heterologen Gen" ist der kodierende Bereich eines Strukturgens zu verstehen, der entweder nicht unter der Kontrolle des eigenen (homologen) Promotors steht oder nicht in dem Organismus, aus dem er abgeleitet ist, exprimiert wird, oder weder unter der Kontrolle des eigenen Promotors noch im ursprünglichen Organismus exprimiert wird.

Das erfindungsgemäße DNA-Molekül kann weiterhin eine für ein Signalpeptid kodierende DNA-Sequenz umfassen, die den Export des exprimierten Proteins gewährleistet, wobei die das Signalpeptid kodierende DNA-Sequenz vorzugsweise direkt mit dem zu exprimierenden heterologen Gen verbunden ist. Für die Sekretion und Modifikation vieler eukaryontischer Proteine ist es erforderlich, die Proteinsequenz am N-Terminus mit einer Signalsequenz zu fusionieren, um die Polypeptide in den Sekretionsapparat zu lenken. Hier kommen beispielsweise Komponenten aus dem *S. occidentalis*-Gen GAM1 oder aus einem hormonellen Gen der Krabbe *Carcinus maenas* (CHH) in Betracht, die für die Sekretion von Hirudin erfolgreich verwendet wurden (Weydemann et al., 1995). Vorzugsweise umfasst eine erfindungsgemäße DNA ein Sekretionssignal ausgewählt aus der Gruppe GAM, GAM-kex2, CHH-kex2, MFα-prepro und dem Hühnerlysozymsekretionssignal. Die vorgenannten Sekretionssignale sind dem Fachmann aus dem Stand der Technik bekannt, beispielsweise aus den europäischen Patentschriften EP 394,538 B1, EP 716,705 B1 und der europäischen Patentanmeldung EP 725,822 A1. Ferner kann die erfindungsgemäße DNA ein Terminatorelement umfassen, das Signalstrukturen für die RNA-Polymerase enthält, die zur Termination der Transkription führen. Beispiele für verwendbare Terminatorelemente sind der *MOX-*oder *PHO1*-Terminator von *H. polymorpha*.

Gegenstand der Erfindung ist ferner eine Wirtszelle, die mindestens eine erfindungsgemäße DNA enthält, wobei die Wirtszelle eine prokaryontische oder eine eukaryontische Zelle ist.

Beispielsweise kann die eukaryontische Zelle eine Pflanzenzelle sein. Vorzugsweise ist die Zelle eine Pilzzelle, besonders bevorzugt eine Hefezelle. Als Wirtszelle zur Ausführung der vorliegenden Erfindung kommen beispielsweise filamentöse Pilze wie etwa *Aspergillus, Neurospora, Mucor, Trichoderma, Acremonium, Sordaria* und *Penicillium* oder Hefen wie *Saccharomyces, Hansenula, Pichia, Torulopsis, Kluyveromyces, Schwanniomyces, Yarrowia, Arxula, Trichosporon* und *Candida* in Betracht.

Ganz besonders bevorzugt ist eine methylotrophe Hefezelle, insbesondere eine solche der Gattung *Candida, Torulopsis, Hansenula* oder *Pichia*. Am meisten bevorzugt ist *Hansenula polymorpha*.

Die vorliegende Erfindung betrifft auch Expressionsvektoren, vorzugsweise Plasmide, bei denen der Vektor eine erfindungsgemäße DNA umfasst.

In einer bevorzugten Ausführungsform enthalten solche Expressionsvektoren das Phytasegen oder *lacZ* als Reporter-Leseraster.

Die vorliegende Erfindung stellt ausserdem einen Kit bereit, umfassend:
(a) einen erfindungsgemäßen Expressionsvektor, der zum Aufnehmen einer DNA geeignet ist, welche ein rekombinantes Protein kodiert, und
(b) eine zum Herstellen des rekombinanten Proteins geeignete Wirtszelle.

Die Aufnahme der für ein rekombinantes Protein kodierenden DNA kann durch alle im Stand der Technik bekannten Klonierungsmethoden erfolgen. Diese werden jedoch nicht alle im Einzelnen beschrieben, weil sie zum selbstverständlichen Handwerkszeug des Fachmanns gehören.

Ferner wird ein Kit bereitgestellt, umfassend
(a) einen erfindungsgemäßen Expressionsvektor, der zum Aufnehmen einer DNA geeignet ist, welche ein rekombinantes Protein kodiert, und
(b) eine Wirtszelle, die zum Herstellen des rekombinanten Proteins unter der Transkriptionskontrolle der Promotorsequenz geeignet ist.

Die erfindungsgemäßen DNA-Moleküle, Wirtszellen, Expressionsvektoren und Kits können zur rekombinanten Expression eines Gens unter der Kontrolle der Promotorsequenz zum Herstellen von einem oder mehreren Proteinen verwendet werden.

Die vorliegende Erfindung betrifft somit auch die Verwendung der erfindungsgemäßen Gegenstände zum Herstellen von Proteinen.

Unter "rekombinanter Expression in einer geeigneten Wirtszelle" sollen alle im Stand der Technik bekannten Expressionsmethoden in bekannten Expressionssystemen verstanden werden, die hier zum Einsatz kommen könnten. Diese werden jedoch nicht alle im Einzelnen beschrieben, weil sie zum selbstverständlichen Handwerkszeug des Fachmanns gehören.

Gegenstand der Erfindung ist ferner ein Verfahren zum Herstellen von einem oder mehreren Proteinen, umfassend:
(i) das Konstruieren eines erfindungsgemäßen Vektors;
(ii) das Einführen des In (i) erhaltenen Expressionsvektors in eine Wirtszelle, die zum Herstellen des rekombinanten Proteins geeignet ist, ggf. nach Induktion der Promotorsequenz;
(iii) das Kultivieren der in (ii) erhaltenen Wirtszelle;
(iv) und ggf. das induzieren der Promotorsequenz in an sich bekannter Weise.

Gegenstand der Erfindung ist auch ein Verfahren zur Herstellung von einem oder mehreren Proteinen, umfassend:
(i) das Einführen eines erfindungsgemäßen Expressionsvektors in eine Wirtszelle, die zum Herstellen des rekombinanten Proteins geeignet ist, ggf. nach Induktion der Promotorsequenz;
(ii) das Kultivieren der in (i) erhaltenen Wirtszelle;
(iii) und ggf. das Induzieren der Promotorsequenz in an sich bekannter Weise.

Ein weiterer Aspekt der vorliegenden Erfindung betrifft Verfahren zum Herstellen der erfindungsgemäßen Expressionsvektoren, wie in Anspruch 20 definiert.

Die Figuren und Beispiele erläutern die Erfindung.

### Figuren

Die Erfindung wird nachfolgend unter Bezugnahme auf die Figuren näher erläutert, wobei die Figuren das Folgende zeigen:

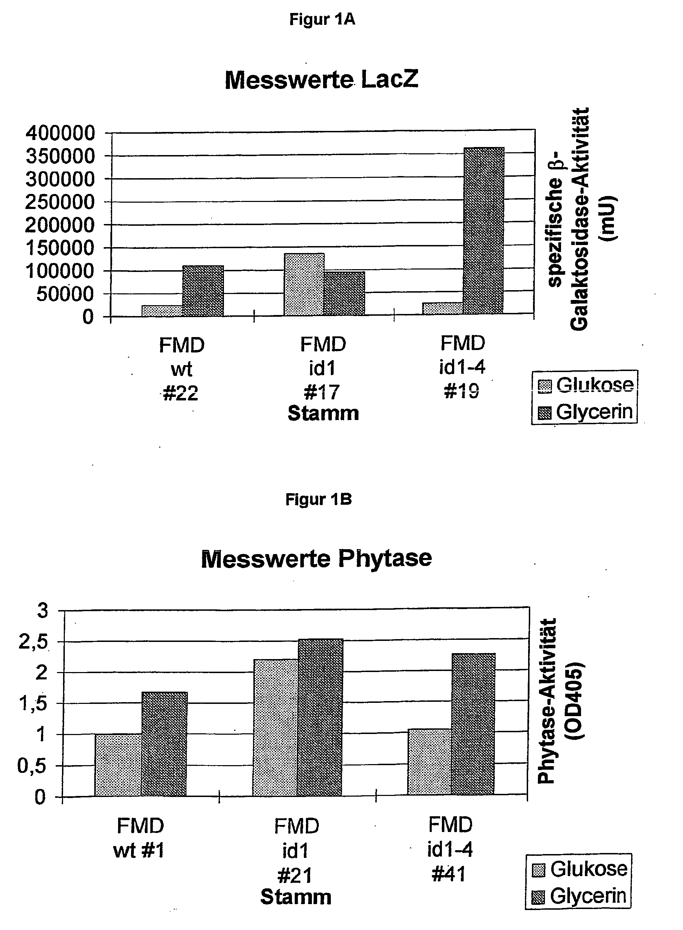

**Figuren 1A** und **1B** zeigen die Expressionseffizienz für Stämme mit den Reportergenen *lacZ* (Figur 1A) und *Phytase* (Figur 1B) unter der Kontrolle der erfindungsgemäßen FMD-Promotor-Varianten (*FMD* id1 und *FMD* id1-4) im graphischen Vergleich mit dem Wildtyp-Promotor (*FMD* wt, Stamm mit Wildtyp-Promotor) nach 24-stündigem Wachstum auf Glukose (hellgrau) oder Glycerin (dunkelgrau).
   Der Wert des *FMD* id1 Promoters nach Glycerininduktion ist zwar sowohl mit *lacZ* als auch mit *Phytase* als Reportergen relativ gering, jedoch absolut gesehen sehr hoch (im Vergleich zum wt *FMD*-Promotor) und zudem ähnlich hoch wie unter Glukosebedingungen. Dies deutet darauf hin, daß es sich bei *FMD* id1 um einen bezüglich der Kohlenstoffquellen Glycerin und Glukose konstitutiven Promotor handelt. *FMD* id1-4 weist auf Glukose ähnliche Werte auf wie der wt *FMD* Promoter, zeigt jedoch auf Glycerin deutlich höhere Werte auf. Sowohl *FMD* id1 als auch *FMD* id1-4 zeigen somit eine gegenüber dem wt *FMD*-Promotor veränderte Regulation und Stärke.

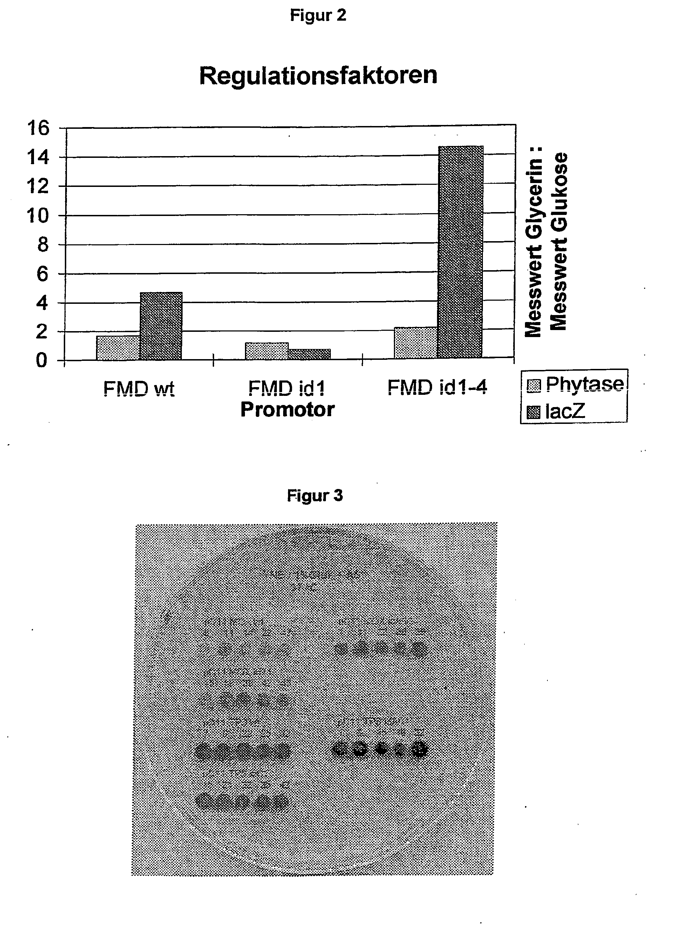

**Figur 2** zeigt die Regulationsfaktoren (der Quotient der Aktivitätsmesswerte auf Glycerin und Glukose) der verschiedenen *FMD*-Promotor-Varianten id1 und id1-4 mit *lacZ* (dunkelgrau) oder *Phytase* (hellgrau) als Reportergen im Vergleich zum Regulationsfaktor des Wildtyp-*FMD*-Promotors (*FMD* wt). Ein Quotient von 1 bedeutet, daß der Promoter bezüglich der untersuchten Kohlenstoffquellen konstitutiv ist. Werte >1 zeigen das Ausmaß Glycerinderepression an. Verglichen mit dem wt *FMD*-Promoter ist der *FMD* id1-Promoter also konstitutiv, während der *FMD* id1-4-Promoter stärker dereprimierbar ist.
**Figur 3** zeigt einen X-Gal-Überschichtungstest mit Stämmen, die zuvor bei 37°C auf YNB/Glukose-Platten gewachsen waren. Sowohl mit dem *MOX*-Plasmid pC11 *MOX* idAC1 als auch mit dem *TPS1*-Promotor zeigte sich, dass die Palindromisierung des ersten (der TATA-Box am nächsten liegenden) ATF/CREB-Sequenz-ähnlichen Motivs unter der getesteten Bedingung zu einer sichtbaren Steigerung der Promotor-Aktivität führte, verglichen mit dem jeweiligen Wildtyp-Promotor (Plasmid pC11 *MOX* wt, Plasmid pC11 *TPS* wt).
   Mix-Promotor
   - Plasmid pC11 MOX idAC1: Natürliche (kryptische) AC1-Sequenz (atggtgtcat) durch zwei Austausche zu atgacgtcat idealisiert (ATF/CREB Bindungsstelle). Die veränderten Basen sind (auch in den folgenden Beispielen) unterstrichen.
   - Plasmid pC11 *MOX* idY1: Natürliche (kryptische) Y1-Sequenz (cgaatgtaat) durch vier Austausche zu attacgtaat idealisiert (YAP Bindungsstelle).

   *TPS1*-Promotor:
   - Plasmid pC11 *TPS* idAC1: Natürliche (kryptische) AC1-Sequenz (atggtgagat) durch vier Austausche zu atgacgtcat idealisiert (ATF/CREB Bindungsstelle).
   - Plasmid pC11 TPS idC1: Natürliche (kryptische) C1-Sequenz (attgaccaat) durch zwei Austausche zu attgcgcaat idealisiert (C/EBP Bindungsstelle)]
**Figuren 4 bis 6****:** In den Figuren 4 bis 6 werden die TATA-Box und einzelne kryptische Bindungsstellen wie folgt dargestellt.
   - Zweifach unterstrichen: Vermeintliche TATA-BOX.
   - Eingerahmt mit durchgehender Linie: mögliche kryptische ATF/CREB-Bindungsstellen für bZip-Proteine.
   - Eingerahmt mit gestrichelter Linie: mögliche kryptische YAP-Bindungsstellen für bZip-Proteine.
   - (AC = kryptische ATF/CREB-Bindungsstelle; Y = kryptische YAP-Bindungsstelle; C = kryptische C/EBP-Bindungsstelle). - Die Basen mit Homologie zur Konsensus-Sequenz sind unterstrichen.
   - Die Numerierung bezieht sich auf den Translationsstart. Alle angegebenen Sequenzen enden mit Guanin. Dieses Guanin ist jeweils die erste Base einer EcoRI-Schnittstelle gaattc. Direkt anschließend folgt das jeweilige Startcodon. Bezogen auf das atg hat besagtes Guanin somit die Nummer (-6).
**Figur 4** zeigt die Sequenz des in den Beispielen verwendeten Wildtyp-*FMD*-Promotors mit vermuteter TATA-BOX und möglichen kryptischen Bindungsstellen für bZip-Proteine.
   Verändert wurden die mit durchgehenden Linien eingerahmten Stellen 1 bis 4, die alle kryptische Varianten der ATF/CREB-Sequenz sind. Die idealisierten Varianten wurden mit id1 bis id4 bezeichnet.
   AC: Kryptische ATF/CREB-Sequenz.
   id# Die kryptische ATF/CREB-Sequenz mit der jeweiligen Nummer wurde in eine ideale ATF/CREB-Sequenz überführt.

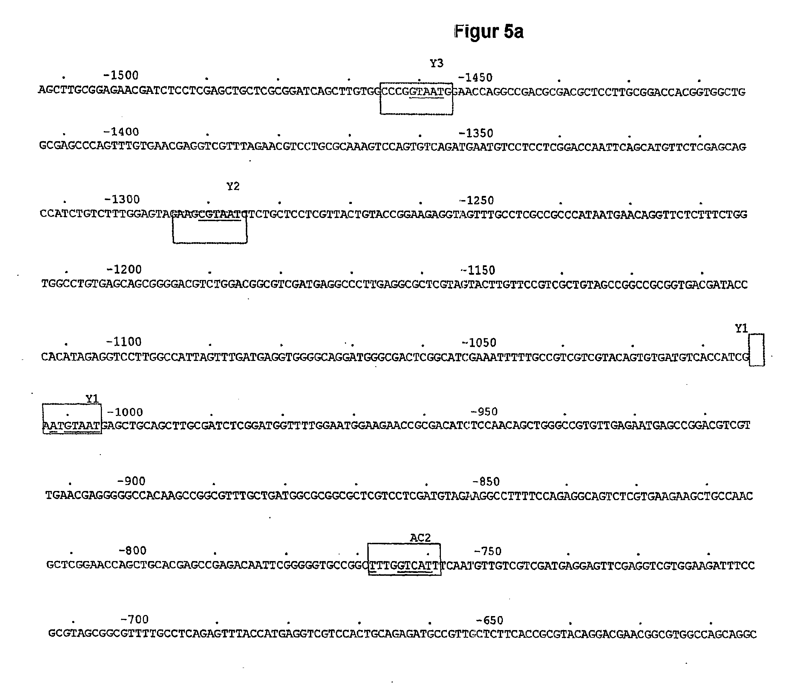

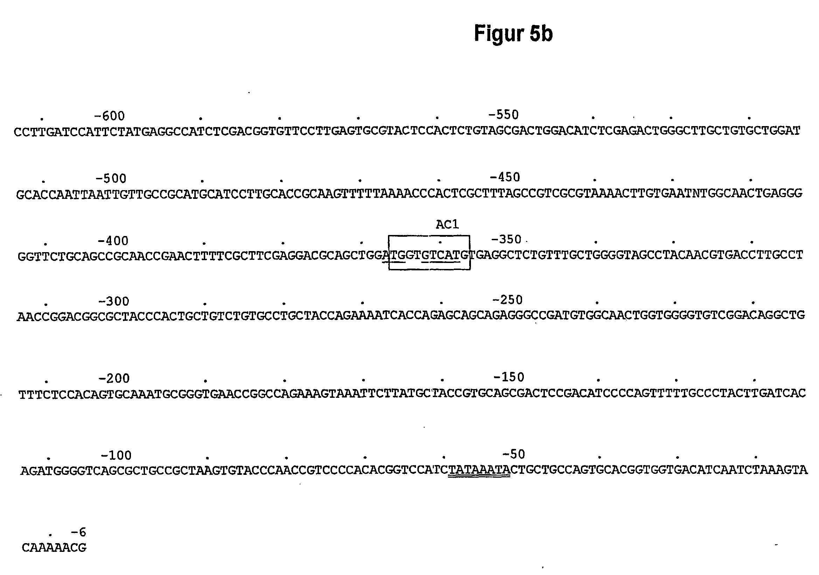

**Figur 5** zeigt die Sequenz des in den Beispielen verwendeten Wildtyp-MOX Promotors mit vermuteter TATA-BOX und möglichen kryptischen Bindungsstellen für bZip-Proteine.
   Verändert wurden die mit durchgehender Linie eingerahmte Stelle 1 (idAC1) und die mit kurzgestrichelter Linie eingerahmte Stelle 1 (idY1).
   AC: Kryptische ATF/CREB-Sequenz.
   idAC# Die kryptische ATF/CREB-Sequenz mit der jeweiligen Nummer wurde in eine ideale ATF/CREB-Sequenz überführt.

   Y: Kryptische YAP-Bindungsstelle.
   idY# Die kryptische YAP-Bindungsstelle mit der jeweiligen Nummer wurde in eine ideale YAP-Bindungsstelle überführt.

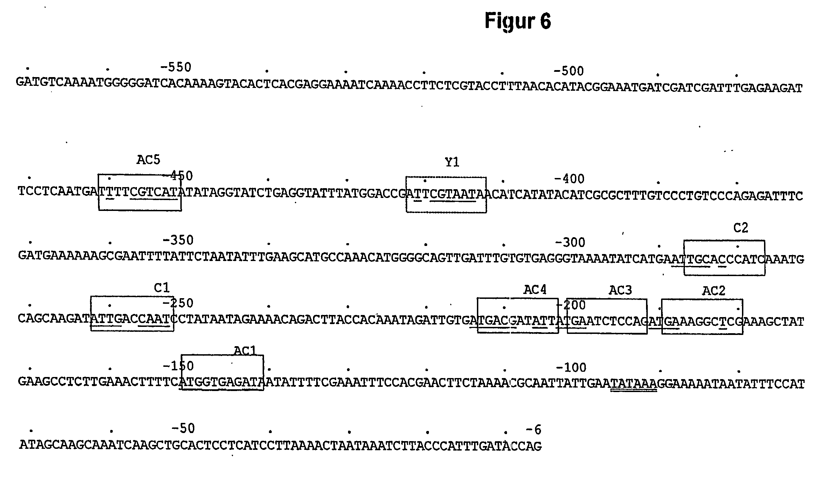

Figur 6 zeigt die Sequenz des in den Beispielen verwendeten Wildtyp-TPS9-Promotors mit vermuteter TATA-BOX und möglichen kryptischen Bindungsstellen für bZip-Proteine.
   Verändert wurden die mit durchgehender Linie eingerahmte Stelle 1 (idAC1) und die mit kurzgestrichelter Linie eingerahmte Stelle 1 (idC1).
   AC: Kryptische ATF/CREB-Sequenz.
   idAC# Die kryptische ATF/CREB-Sequenz mit der jeweiligen Nummer wurde in eine ideale ATF/CREB-Sequenz überführt.

   C: Kryptische C/EBP-Bindungsstelle.
   idC# Die kryptische C/EBP-Bindunasstelle mit der jeweiligen Nummer wurde in eine ideale C/EBP-Bindungsstelle überführt.
**Figuren 7A, 7B** **und** **7C** zeigen Genkarten der *lacZ*-Reporterplasmide pC11-*FMD*, pC11-**MOX** und pC11-*TPS1*, die sich nur durch den jeweiligen, im Plasmidnamen angegebenen Promotor unterscheiden.
**Figur 8** zeigt ideale Bindungsstellen für bZip-Proteine, wobei die erste Spalte die Bezeichnung, die mittlere Spalte die idealisierte Sequenz und die rechte Spalte den biologischen Ursprung der Sequenz wiedergibt.

### Beispiele

### Einleitung

Die folgenden Beispiele zeigen die Veränderung der Transkriptionseffizienz von Promotoren methylotropher Hefen wie die der *FMD*-, *MOX-* und *TPS1*-Promotoren durch Überführung einzelner oder mehrerer kryptischer DNA-Bindungsstellen in ihre palindromischen und somit formal idealisierten Derivate.

### Darstellung veränderter Wildtyp-Promotoren

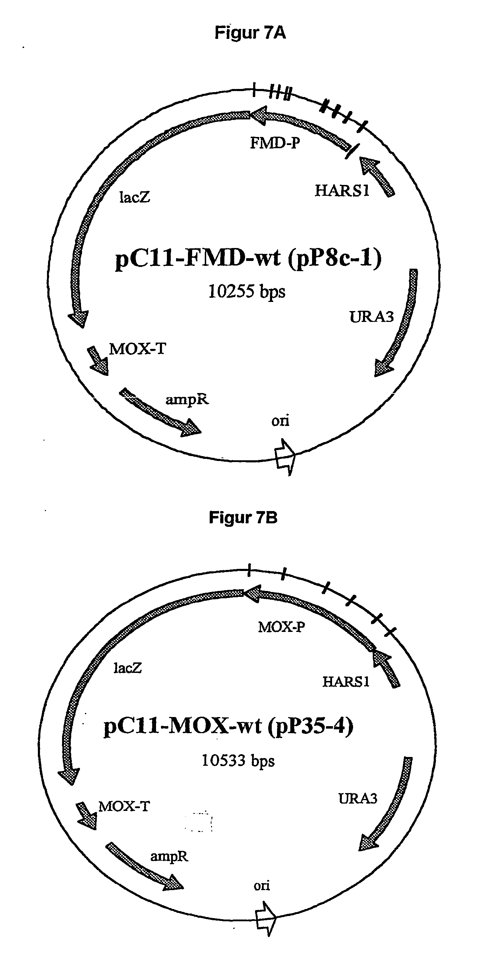

Die zu verändernden Wildtyp-Promotoren (siehe Figuren 4 (*FMD*), 5 (*MOX*), und 6 (*TPS1*)) wurden in pUC18 überführt und dort mittels PCR-Mutagenese in an sich bekannter Weise gezielt verändert. Nach Verifikation der Austausche durch Sequenzanalyse wurden die veränderten Promotoren in Expressionsplasmide subkloniert, die entweder *Phytase* oder *lacZ* als Reporter-Leseraster enthielten. Die auf diese Weise erzeugten zwei Serien von Reporterplasmiden unterscheiden sich nur in ihren Promotorsequenzen (siehe beispielsweise für /*acZ* als Reportergen die Figuren 7A (*FMD-*Promotor), Figur 7B (*MOX*-Promotor) und Figur 7C (*TPS1*-Promotor)).

### Integratives Testsystem

Um Promotoren objektiv miteinander vergleichen zu können, müssen die Reportergendosen der zu vergleichenden Stämme vergleichbar sein. Es wurde daher das folgende integrative Testsystem konstruiert, welches sowohl für *lacZ-* als auch für *Phytase-*Reporterplasmide geeignet ist. *H. polymorpha* wurde mit dem jeweiligen Reporterplasmid transformiert und pro Konstrukt wurden 48 Stämme mit genomisch integrierter Plasmid-DNA erzeugt Diese jeweils 48 Stämme wurden anschließend einem Reporterprotein-Test unterzogen. Dieser Test lieferte zwei Informationen: zum einen die Konstrukt-bedingten Reportergen-Expressionsstärken unter der getesten Bedingung als Endpunktbestimmung, zum anderen die Identifikation der jeweils zwei Stämme mit den höchsten Reportergen-Expressionsraten. Diese Stämme wurden dann weiteren vergleichenden Experimenten unterzogen. Desweiteren wurde bei ihnen die Kopienzahl des integrierten Reporterplasmids bestimmt, um auszuschließen, dass etwaige verstärkte Promotoraktivitäten auf einen Gendosis-Effekt zurückzuführen sind.

### Bestimmung von FMD-Promotor-Varianten mit erhöhter Aktivität und/oder veränderter Regulation

Die Stämme mit id1 oder id1-4 in der Stämmbezeichnung enthalten Expressionskassetten mit *FMD*-Promotorvarianten mit gezielten Veränderungen im Bereich kryptischer bZip-Bindungsstellen (hier: im Bereich der ATF/CREB-Sequenzen 1 bis 4). Die verschiedenen Promotoren wurden sowohl in Kombination mit *lacZ* als auch in Kombination mit *dem Phytasegen* getestet.

Für die Quantifizierung der ß-Galaktosidase wurden ONPG-Messungen (ONPG: ortho-Nitrophenyl-β-D-Galaktopyranosid; J. H. Miller (1972) Experiments in Molecular Genetics. Cold Spring Harbour Laboratory Press. Cold Spring Harbour, NY, USA) mit intrazellulären löslichen Fraktionen durchgeführt.

Für Phytase wurde der Phytase-Test mit Kulturüberständen durchgeführt (A F. Mayer, K. Hellmuth, H. Schlieker, R. Lopez-Ulibarry, S. Oertel, U. Dahlems, A. W. M. Strasser, A. P. G. M. van Loon (1999) An expression system matures: a highly efficient and cost-effective process for phytase production by recombinant strains of Hansenula polymorpha. Biotechnology and Bioengeneering 63, 373-381; L. Pasamontes, M. Haiker, M. Wyss, M. Tessier, A. P. G. M. van Loon (1997) Gene cloning, purification and characterization of a heat-stable phytase from the fungus Aspergillus fumigatus. Appl. Environ. Microbiol. 63, 1696-1700).

Es wurden unabhängig vom Reportergen zwei unterschiedliche Promotor-Merkmale analysiert: Stärke und Regulation. Die Bestimmung der relativen Kopienzahlen der integrierten Plasmide ergab, dass die verglichenen Stämme mit veränderten Promotoren weniger oder gleich viele Kopien enthalten als der jeweils zugrunde liegende Stramm mit dem Wildtyp-*FMD*-Promotor.

### Stärke der veränderten FMD-Promotoren

Zur Beurteilung der Stärke des jeweiligen erfindungsgemäß veränderten *FMD-*Promotors im Vergleich zur Stärke des Wildtyp-*FMD*-Promotors, wurde folgendes Experiment durchgeführt. Der jeweils stärkste Stamm pro Reporterplasmid wurde für 48 Stunden auf Glukose oder auf Glycerin als Kohlenstoffquelle kultiviert. Nach 4, 24 und 48 Stunden wurden Zellen (*lacZ*) oder Kulturüberstand (*Phytasegen*) geerntet. Für *lacZ* wurden, ausgehend von den geernteten Zellen, intrazelluläre lösliche Fraktionen präpariert, mit denen anschließend eine ONPG-Messung zur Bestimmung der spezifischen β-Galaktosidase-Aktivität durchgeführt wurde. Für Phytase wurde ein Phosphatase-Test durchgeführt, in dem die geetnteten Kulturüberstände direkt eingesetzt wurden. Figuren 1 A und B zeigen die absoluten Meßwerte der getesteten Stämme nach 24-stündigem Wachstum auf Glukose (Figur 1A) und Glycerin (Figur 1 B). Sowohl mit *lacZ* als auch mit *Phytasegen* werden mit den veränderten Promotoren Spitzenwerte erreicht, die deutlich über den mit dem Wildtyp-*FMD*-Promotor erzielten Werten liegen. Dieser Effekt war mit /*acZ* (Abb. 1B) generell stärker ausgeprägt als mit *Phytasegen* (Abb. 1A). Dies kommt dadurch zustande, dass die Phytase ein sekretorisches Glyco-Protein ist, bei dem zwischen der Transkription des Gens und dem Auftreten des aktiven Genprodukts im Kulturüberstand eine Vielzahl von Transport- und Modifikationsschritten absolviert werden müssen, die für die Bereitstellung der cytosolisch lokalisierten ß-Galaktosidase nicht erforderlich sind. Sowohl mit *Phytasegen* als auch mit /*acZ* erreichen einzelne *FMD-*Promotor-Varianten auf Glukose höhere Aktivitäten als der Wildtyp-Promotor auf Glycerin. Im Falle von /*acZ* wurden die mit dem Wildtyp-Promotor erzielten Spitzenwerte um das bis zu 3,5-fache übertroffen, im Fall von *Phytasegen* um das bis zu 2,5-fache (siehe Figuren 1A und 1B).

### Regulation von veränderten FMD-Promotoren

Die getesteten *FMD*-Promotor-Varianten zeigten teilweise eine von der des Wildtyp-Promotors abweichende Regulation. Als Maß für die Regulation wurde der Quotient aus den Messwerten nach 24-stündiger Kultivierung auf Glycerin und auf Glukose gebildet Ein Faktor von 1 bedeutet Konstitutivität, bezogen auf die untersuchten Kohlenstoffquellen. Höhere Faktoren zeigen an, dass der jeweilige Promotor auf Glycerin eine höhere Aktivität aufweist als auf Glukose. Die Abfolge der mit den verschiedenen *FMD-*Promotor-Varianten gemessenen Regulationsfaktoren ist mit beiden Reportergenen (*lacZ* und *Phytasegen*) qualitativ identisch (Figur 2): id1-4 > wt > id1.

Der Promotor id1 ist annähernd konstitutiv, während der Promotor id1-4 mit beiden Reportergenen einen höheren Regulationsfaktor aufweist als der Wildtyp-*FMD*-Promotor (siehe Figur 2).

Somit wurde gezeigt, dass erfindungsgemäß sowohl die Stärke, als auch die Regulation von methylotrophen Promotoren, hier dem *FMD*-Promotor signifikant verändert werden können.

### MOX- und TPS1-Promotor-Varianten mit erhöhter Aktivität auf Medien mit Glukose als Kohlenstoffquelle

Zum Nachweis der Übertragbarkeit der für *FMD*-Promotoren oben gezeigten Modulierbarkeit der Effizienz auf andere Promotoren methylotropher Hefen wurden auch ein *MOX*-Promotor und ein *TPS1*-Promotor erfindungsgemäß in ihrer Transkriptionseffizienz moduliert. In der Sequenz des *MOX*-Promotors von *H. polymorpha* wurden fünf kryptische Bindungsstellen für bZip-Proteine identifiziert, und zwar zwei Motive mit Ähnlichkeit mit der ATF/CREB-Sequenz und drei Motive mit Ähnlichkeit mit der YAP-Bindungsstelle (siehe Figur 5). In Analogie zu den am *FMD*-Promotor durchgeführten Veränderungen wurden zwei dieser Sequenzmotive palindromisiert. Die resultierenden Promotor-Varianten *MOX*-idA/CI und *MOX*-idY1 sowie der Wildtyp-*MOX*-Promotor wurden, wie bereits für den *FMD*-Promotor beschrieben, so in das *lacZ*-Reporterplasmid überführt, dass sie die Kontrolle des Reportergens übernahmen.

In der Sequenz des *TPS1*-Promotors wurden insgesamt acht mögliche kryptische bZip-Motive identifiziert (siehe Figur 6). Von den in diesem Beispiel gezeigten Motiven haben fünf Ähnlichkeit mit der ATF/CREB-Sequenz, zwei mit der C/EBP-Bindungsstelle und eines mit der YAP-Bindungsstelle. Für diesen Promotor wurden zwei Varianten mit je einem palindromisierten Sequenzmotiv konstruiert, *TPS*-idA/C1 und *TPS*-idC1.

Weitere kryptische bZip oder Zinkfinger-Motive sind dem Fachmann durch eingehendere Analyse zugänglich, z. B. unter Inanspruchnahme EDV-unterstützter Vergleichsalgorithmen.

Pro Konstrukt wurden 48 Stämme generiert. Wie für die *FMD*-Konstrukte beschrieben, wurden mittels eines X-Gal-Überschichtungstest zunächst die Stämme mit den höchsten Reportergen-Aktivitäten ermittelt, die dann detaillierter analysiert wurden. Figur 3 zeigt einen X-Gal-Überschichtungstest von je fünf dieser Stämme, die zuvor bei 37°C auf YNB/Glukose-Platten gewachsen waren. Sowohl mit dem *MOX*- als auch bei dem *TPS1*-Promotor zeigte sich, dass die Palindromisierung des ersten (der TATA-Box am nächsten liegenden) ATF/CREB-Sequenz-ähnlichen Motivs unter der getesteten Bedingung zu einer sichtbaren Steigerung der Promotor-Aktivität führte, verglichen mit dem jeweiligen Wildtyp-Promotor (siehe Figur 3). Mit anderen getesteten Kohlenstoffquellen konnte dieser Effekt nicht beobachtet werden. Dieses Ergebnis zeigt, dass auch die Transkriptionseigenschaften von *MOX*- und TPS1-Promotor durch die Veränderung, insbesondere Palindromisierung bereits vorhandener, kryptischer DNA-Bindungsstellen verändert werden können.

## Patentansprüche

1. DNA, umfassend mindestens eine Promotorsequenz, die von einem Wildtyp-Promotor aus *Hansenula polymorpha,* der ausgewählt ist aus der Gruppe bestehend aus dem MOX-, FMD- und TPS1-Promotor, abgeleitet ist, **dadurch gekennzeichnet, dass** die Transkriptionseffizienz dieser Promotorsequenz im Vergleich zu der Effizienz des Wildtyp-Promotors durch Verändern einer DNA-Bindungsstelle moduliert ist, wobei das Verändern durch Palindromisierung mindestens einer der DNA-Sequenzen gemäß SEQ ID No: 1-4, 11, 13, 16 und 20 erfolgt, wobei die palindromisierte Sequenz nicht mehr als 2 Basen pro Strang von einer vollständig palindromisierten Sequenz abweicht.

2. DNA nach Anspruch 1, **dadurch gekennzeichnet, dass** die Transkriptionseffizienz der Promotorsequenz im Vergleich zu der Effizienz des Wildtyp-Promotors erhöht ist.

3. DNA nach Anspruch 1, **dadurch gekennzeichnet, dass** die Transkriptionseffizienz der Promotorsequenz im Vergleich zu der Effizienz des Wildtyp-Promotors um mindestens den Faktor 1,5, vorzugsweise um mindestens den Faktor 2, besonders bevorzugt um mindestens den Faktor 2,5 erhöht ist.

4. DNA nach Anspruch 1, **dadurch gekennzeichnet, dass** die Transkriptionseffizienz der Promotorsequenz im Vergleich zu der Effizienz des Wildtyp-Promotors gemindert ist, vorzugsweise um mindestens den Faktor 0,5 gemindert.

5. DNA nach einem der Ansprüche 1 bis 4, **dadurch gekennzeichnet, dass** die Promotorsequenz durch die Insertion, Deletion und/oder den Austausch mindestens einer Base, vorzugsweise durch den Austausch von ein oder zwei Basen, von einem Wildtyp-Promotor abgeleitet ist.

6. DNA nach einem der Ansprüche 1 bis 5, **dadurch gekennzeichnet, dass** die palindromisierte Sequenz symmetrisch oder unsymmetrisch, vorzugsweise symmetrisch ist und in nur einer Base und besonders bevorzugt gar nicht von einer vollständig palindromen Sequenz abweicht.

7. DNA gemäß einem der vorangegangenen Ansprüche, **dadurch gekennzeichnet, dass** sie weiterhin mindestens eine unter der Transkriptionskontrolle der Promotorsequenz stehende DNA-Sequenz für ein homologes und/oder heterologes Gen umfasst.

8. DNA gemäß einem der vorangegangenen Ansprüche, **dadurch gekennzeichnet, dass** sie weiterhin eine für ein Sekretionssignal kodierende DNA-Sequenz umfasst.

9. DNA gemäß einem der vorangegangenen Ansprüche, **dadurch gekennzeichnet, dass** das Sekretionssignal ausgewählt ist aus der Gruppe GAM, GAM-kex2, CHH- kex2, MFα-prepro und dem Hühnerlysozymsekretionssignal.

10. Wirtszelle, **dadurch gekennzeichnet, dass** die Wirtszelle mindestens eine DNA nach einem der Ansprüche 1 bis 9 enthält.

11. Wirtszelle nach Anspruch 10, **dadurch gekennzeichnet, dass** die DNA in das Genom der Wirtszelle integriert ist oder als extrachromosomales DNA-Molekül, vorzugsweise episomal, in der Wirtszelle vorliegt.

12. Wirtszelle nach einem der Ansprüche 10 oder 11, **dadurch gekennzeichnet, dass** die Wirtszelle eine Hefezelle, vorzugsweise eine methylotrophe Hefezelle, besonders bevorzugt eine Hefezelle der Gattungen *Candida*, *Torulopsis*, *Hansenula* oder *Pichia*, besonders bevorzugt *Hansenula polymorpha* ist.

13. Expressionsvektor, vorzugsweise ein Plasmid, **dadurch gekennzeichnet, dass** der Vektor eine DNA nach einem der Ansprüche 1 bis 9 umfasst.

14. Expressionsvektor nach Anspruch 13, **dadurch gekennzeichnet, dass** der Vektor das Phytasegen oder lacZ als Reporter-Leseraster enthält.

15. Kit, umfassend:
(a) einen Expressionsvektor nach Anspruch 13 oder 14, der zum Klonieren einer DNA geeignet ist, welche ein rekombinantes Protein kodiert, und
(b) eine zum Herstellen des rekombinanten Proteins geeignete Wirtszelle.

16. Verwendung einer DNA nach einem der Ansprüche 1 bis 9 oder einer Wirtszelle nach einem der Ansprüche 10 bis 12 oder eines Expressionsvektors nach Anspruch 13 oder 14 oder eines Kits nach Anspruch 15 zur Expression eines Gens.

17. Verwendung einer DNA nach einem der Ansprüche 1 bis 9 oder einer Wirtszelle nach einem der Ansprüche 10 bis 12 oder eines Expressionsvektors nach Anspruch 13 oder 14 oder eines Kits nach Anspruch 15 zum Herstellen von einem oder mehreren Proteinen.

18. Verfahren zum Herstellen von einem oder mehreren Proteinen, umfassend:
(i) Einführen eines Expressionsvektors nach Anspruch 13, enthaltend eine DNA nach Anspruch 7, in eine Wirtszelle, die zum Herstellen des rekombinanten Proteins geeignet ist, ggf. nach Induktion der Promotorsequenz;
(ii) Kultivieren der in (i) erhaltenen Wirtszelle;
(iii) und ggf. Induzieren der Promotorsequenz in an sich bekannter Weise, wodurch das oder die Protein(e) von der Wirtszelle hergestellt wird/werden.

19. Verfahren zum Herstellen von einem oder mehreren Proteinen, umfassend:
(i) Konstruieren des Expressionsvektors, enthaltend eine DNA nach Anspruch 7;
(ii) Einführen eines Expressionsvektors in eine Wirtszelle, die zum Herstellen eines rekombinanten Proteins geeignet ist, ggf. nach Induktion der Promotorsequenz;
(iii) Kultivieren der in (ii) erhaltenen Wirtszelle;
(iv) und ggf. Induzieren der Promotorsequenz in an sich bekannter Weise, wodurch das oder die Protein(e) von der Wirtszelle hergestellt wird/werden.

20. Verfahren zur Herstellung von Expressionsvektoren gemäß einem der Ansprüche 13 oder 14, umfassend:
(i) das Einfügen einer DNA gemäß einem der Ansprüche 1 bis 9 in einen Expressionsvektor; oder
(ii) die Veränderung einer Promotorsequenz aus *Hansenula polymorpha*, wobei der Promotor ausgewählt ist aus der Gruppe bestehend aus dem MOX-, FMD- und TPS1-Promotor, in einem Expressionsvektor durch Insertion, Deletion und/oder Austausch mindestens eines Basenpaares in an sich bekannter Weise, wobei die Transkriptionseffizienz dieser Promotorsequenz im Vergleich zu der Effizienz des Wildtyp-Promotors durch Verändern einer DNA-Bindungsstelle moduliert ist, wobei das Verändern durch Palindromisierung mindestens einer der DNA-Sequenzen gemäß SEQ ID No: 1-4, 11, 13, 16 und 20 erfolgt und wobei die palindromisierte Sequenz nicht mehr als 2 Basen pro Strang von einer vollständig palindromisierten Sequenz abweicht; und
(iii) gegebenenfalls die Einführung eines heterologen Gens in an sich bekannter Weise in einen Expressionsvektor, so dass das heterologe Gen unter der Transkriptionskontrolle der DNA aus (a) oder (b) steht.

## Claims

1. DNA comprising at least a promoter sequence which is derived from a wild-type promoter from *Hansenula polymorpha* chosen from the group consisting of the MOX, FMD and TPS1 promoter, **characterized in that** the transcription efficiency of this promoter sequence is modulated compared with the efficiency of the wild-type promoter by modification of a DNA binding site, wherein the modification is effected by palindromization of at least one of the DNA sequences according to SEQ ID No: 1-4, 11, 13, 16 and 20, wherein the palindromized sequence deviates from a completely palindromized sequence by not more than 2 bases per strand.

2. DNA according to claim 1, **characterized in that** the transcription efficiency of the promoter sequence is increased compared with the efficiency of the wild-type promoter.

3. DNA according to claim 1, **characterized in that** the transcription efficiency of the promoter sequence is increased by at least the factor 1.5, preferably by at least the factor 2, particularly preferably by at least the factor 2.5, compared with the efficiency of the wild-type promoter.

4. DNA according to claim 1, **characterized in that** the transcription efficiency of the promoter sequence is decreased compared with the efficiency of the wild-type promoter, preferably decreased by at least the factor 0.5.

5. DNA according to one of claims 1 to 4, **characterized in that** the promoter sequence is derived from a wild-type promoter by the insertion, deletion and/or the substitution of at least one base, preferably by the substitution of one or two bases.

6. DNA according to one of claims 1 to 5, **characterized in that** the palindromized sequence is symmetric or unsymmetric, preferably symmetric, and deviates from a completely palindromic sequence in only one base, and particularly preferably not at all.

7. DNA according to one of the preceding claims, **characterized in that** it furthermore comprises at least one DNA sequence, which is under the transcription control of the promoter sequence, for a homologous and/or heterologous gene.

8. DNA according to one of the preceding claims, **characterized in that** it furthermore comprises a DNA sequence which codes for a secretion signal.

9. DNA according to one of the preceding claims, **characterized in that** the secretion signal is chosen from the group of GAM, GAM-kex2, CHH-kex2, MFα-prepro and the chicken lysozyme secretion signal.

10. Host cell, **characterized in that** the host cell comprises at least one DNA according to one of claims 1 to 9.

11. Host cell according to claim 10, **characterized in that** the DNA is integrated into the genome of the host cell or is present in the host cell as an extrachromosomal DNA molecule, preferably episomally.

12. Host cell according to one of claims 10 or 11, **characterized in that** the host cell is a yeast cell, preferably a methylotrophic yeast cell, particularly preferably a yeast cell of the genera *Candida*, *Torulopsis, Hansenula* or *Pichia*, particularly preferably *Hansenula polymorpha*.

13. Expression vector, preferably a plasmid, **characterized in that** the vector comprises a DNA according to one of claims 1 to 9.

14. Expression vector according to claim 13, **characterized in that** the vector comprises the phytase gene or lacZ as a reporter reading frame.

15. Kit comprising:
(a) an expression vector according to claim 13 or 14, which is suitable for cloning a DNA which codes for a recombinant protein, and
(b) a host cell which is suitable for production of the recombinant protein.

16. Use of a DNA according to one of claims 1 to 9 or of a host cell according to one of claims 10 to 12 or of an expression vector according to claim 13 or 14 or of a kit according to claim 15 for expression of a gene.

17. Use of a DNA according to one of claims 1 to 9 or of a host cell according to one of claims 10 to 12 or of an expression vector according to claim 13 or 14 or of a kit according to claim 15 for production of one or more proteins.

18. Method for the production of one or more proteins, comprising:
(i) introduction of an expression vector according to claim 13, comprising a DNA according to claim 7, into a host cell which is suitable for the production of the recombinant protein, optionally after induction of the promoter sequence;
(ii) cultivation of the host cell obtained in (i);
(iii) and optionally induction of the promoter sequence in a manner known per se, as a result of which the protein(s) is/are produced by the host cell.

19. Method for the production of one or more proteins, comprising:
(i) construction of the expression vector, containing a DNA according to claim 7;
(ii) introduction of an expression vector into a host cell which is suitable for the production of a recombinant protein, optionally after induction of the promoter sequence;
(iii) cultivation of the host cell obtained in (ii);
(iv) and optionally induction of the promoter sequence in a manner known per se, as a result of which the protein(s) is/are produced by the host cell.

20. Method for the production of expression vectors according to one of claims 13 or 14, comprising:
(i) the insertion of a DNA according to one of claims 1 to 9 into an expression vector; or
(ii) the modification of a promoter sequence from *Hansenula polymorpha*, wherein the promoter is chosen from the group consisting of the MOX, FMD and TPS1 1 promoter, in an expression vector by insertion, deletion and/or substitution of at least one base pair in a manner known per se, wherein the transcription efficiency of this promoter sequence is modulated compared with the efficiency of the wild-type promoter by modification of a DNA binding site, wherein the modification is effected by palindromization of at least one of the DNA sequences according to SEQ ID No: 1-4, 11, 13, 16 and 20, and wherein the palindromized sequence deviates from a completely palindromized sequence by not more than 2 bases per strand; and
(iii) optionally the introduction of a heterologous gene into an expression vector in a manner known per se, so that the heterologous gene is under the transcription control of the DNA from (a) or (b).

## Revendications

1. ADN comprenant au moins une séquence promotrice, qui est dérivée d'un promoteur de type sauvage de *Hansenula polymorpha,* choisi dans le groupe constitué par le promoteur MOX, FMD et TPS1, **caractérisé en ce que** l'efficacité de transcription de cette séquence promotrice est modulée par rapport à l'efficacité du promoteur de type sauvage par modification d'un site de liaison de l'ADN, où la modification s'effectue par palindromisation d'au moins une des séquences d'ADN selon SEQ ID N° 1 - 4, 11, 13, 16 et 20, où la séquence palindromisée ne diffère pas d'une séquence entièrement palindromisée par plus de 2 bases par brin.

2. ADN selon la revendication 1, **caractérisé en ce que** l'efficacité de transcription de la séquence promotrice est accrue par rapport à l'efficacité du promoteur de type sauvage.

3. ADN selon la revendication 1, **caractérisé en ce que** l'efficacité de transcription de la séquence promotrice par rapport à l'efficacité du promoteur de type sauvage est accrue au moins d'un facteur 1,5, de préférence au moins d'un facteur 2, de préférence au moins d'un facteur 2,5.

4. ADN selon la revendication 1, **caractérisé en ce que** l'efficacité de transcription de la séquence promotrice par rapport à l'efficacité du promoteur de type sauvage est réduite, de préférence au moins d'un facteur 0,5.

5. ADN selon l'une des revendications 1 à 4, **caractérisé en ce que** la séquence promotrice est dérivée d'un promoteur de type sauvage par insertion, délétion et/ou remplacement d'au moins une base, de préférence par remplacement d'une ou deux bases.

6. ADN selon l'une des revendications 1 à 5, **caractérisé en ce que** la séquence palindromisée est symétrique ou non symétrique, de préférence symétrique, et ne diffère d'une séquence entièrement palindromisée que par une seule base, et particulièrement préférentiellement n'en diffère absolument pas.

7. ADN selon l'une des revendications précédentes, **caractérisé en ce qu'**il comprend également au moins une séquence d'ADN se trouvant sous le contrôle de transcription de la séquence promotrice, pour un gène homologue et/ou hétérologue.

8. ADN selon l'une des revendications précédentes, **caractérisé en ce qu'**il comprend également une séquence d'ADN codant pour un signal de sécrétion.

9. ADN selon l'une des revendications précédentes, **caractérisé en ce que** le signal de sécrétion est choisi dans le groupe constitué par GAM, GAM-kex2, CHH-kex2, MFα-prépro et le signal de sécrétion du lysozyme de poulet.

10. Cellule hôte, **caractérisée en ce que** la cellule hôte comprend au moins un ADN selon l'une des revendications 1 à 9.

11. Cellule hôte selon la revendication 10, **caractérisée en ce que** l'ADN est intégré au génome de la cellule hôte ou se présente sous forme de molécule d'ADN extrachromosomique, de préférence épisomale, dans la cellule hôte.

12. Cellule hôte selon l'une des revendications 10 ou 11, **caractérisée en ce que** la cellule hôte est une cellule de levure, de préférence une cellule de levure méthylotrophe, particulièrement préférentiellement une cellule de levure des genres *Candida*, *Torulopsis*, *Hansenula* ou *Pichia*, particulièrement préférentiellement *Hansenula polymorpha.*

13. Vecteur d'expression, de préférence un plasmide, **caractérisé en ce que** le vecteur comprend un ADN selon l'une des revendications 1 à 9.

14. Vecteur d'expression selon la revendication 13, **caractérisé en ce que** le vecteur comprend le gène de la phytase ou le gène lacZ à titre de cadre de lecture rapporteur.

15. Kit comprenant :
(a) un vecteur d'expression selon la revendication 13 ou 14, qui est adapté pour le clonage d'un ADN, lequel code pour une protéine recombinante, et
(b) une cellule hôte adaptée pour la production de la protéine recombinante.

16. Utilisation d'un ADN selon l'une des revendications 1 à 9 ou d'une cellule hôte selon l'une des revendications 10 à 12 ou d'un vecteur d'expression selon la revendication 13 ou 14 ou d'un kit selon la revendication 15 pour l'expression d'un gène.

17. Utilisation d'un ADN selon l'une des revendications 1 à 9 ou d'une cellule hôte selon l'une des revendications 10 à 12 ou d'un vecteur d'expression selon la revendication 13 ou 14 ou d'un kit selon la revendication 15 pour la production d'une ou de plusieurs protéines.

18. Procédé de production d'une ou de plusieurs protéines, comprenant :
(i) l'introduction d'un vecteur d'expression selon la revendication 13, contenant un ADN selon la revendication 7, dans une cellule hôte, qui est adaptée pour la production de la protéine recombinante, le cas échéant après induction de la séquence promotrice ;
(ii) la culture de la cellule hôte obtenue au (i) ;
(iii) et le cas échéant l'induction de la séquence promotrice par une manière connue en soi, de telle sorte que la ou les protéine(s) est/sont produite(s) par la cellule hôte.

19. Procédé de production d'une ou de plusieurs protéines, comprenant :
(i) la construction du vecteur d'expression, comprenant un ADN selon la revendication 7 ;
(ii) l'introduction d'un vecteur d'expression dans une cellule hôte, qui est adaptée pour la production d'une protéine recombinante, le cas échéant après induction de la séquence promotrice ;
(iii) la culture de la cellule hôte obtenue au (ii) ;
(iv) et le cas échéant l'induction de la séquence promotrice par manière connue en soi, de telle sorte que la ou les protéine(s) est/sont produite(s) par la cellule hôte.

20. Procédé de production de vecteurs d'expression selon l'une des revendications 13 ou 14, comprenant:
(i) l'introduction d'un ADN selon l'une des revendications 1 à 9 dans un vecteur d'expression ; ou
(ii) la modification d'une séquence promotrice de *Hansenula polymorpha,* le promoteur étant choisi dans le groupe constitué par le promoteur MOX, FMD et TPS1, dans un vecteur d'expression par insertion, délétion et/ou remplacement d'au moins une paire de bases par une manière connue en soi, où l'efficacité de transcription de cette séquence promotrice est modulée par rapport à l'efficacité du promoteur de type sauvage par modification d'un site de liaison de l'ADN, la modification se faisant par palindromisation d'au moins une des séquences d'ADN selon SEQ ID N°1 - 4, 11, 13, 16 et 20 et la séquence palindromisée ne différant pas d'une séquence entièrement palindromisée par plus de 2 bases par brin ; et
(iii) le cas échéant l'introduction d'un gène hétérologue par une manière connue en soi dans un vecteur d'expression de sorte que le gène hétérologue soit sous le contrôle de transcription de l'ADN provenant de (a) ou (b).


### SEQUENCE LISTING

<110> RHEIN BIOTECH GESELLSCHAFT FÜR NEUE BIOTECHNOLOGISCHE PROZESSE UND PRODUKT MBH
<120> Promotoren mit veränderter Transkriptionseffizienz
<130> P33924-01912
<160> 26
<170> PatentIn version 3.1
<210> 1
   <211> 10
   <212> DNA
   <213> Hansenula polymorpha
<220>
   <223> Beschreibung der Sequenz: FMD-Promotorabschnitt
<400> 1
   cactggtcac 10
<210> 2
   <211> 10
   <212> DNA
   <213> Hansenula polymorpha
<220>
   <223> Beschreibung der Sequenz: FMD-Promotorabschnitt
<400> 2
   atgactttgg 10
<210> 3
   <211> 10
   <212> DNA
   <213> Hansenula polymorpha
<220>
   <223> Beschreibung der Sequenz: FMD-Promotorabschnitt
<400> 3
   ctgacagggt 10
<210> 4
   <211> 10
   <212> DNA
   <213> Hansenula polymorpha
<220>
   <223> Beschreibung der Sequenz: FMD-Promotorabschnitt
<400> 4
   gtgacggaat 10
<210> 5
   <211> 10
   <212> DNA
   <213> Hansenula polymorpha
<220>
   <223> Beschreibung der Sequenz: FMD-Promotorabschnitt
<400> 5
   atgacagcat 10
<210> 6
   <211> 10
   <212> DNA
   <213> Hansenula polymorpha
<220>
   <223> Beschreibung der Sequenz: FMD-Promotorabschnitt
<400> 6
   tggaggtcaa 10
<210> 7
   <211> 10
   <212> DNA
   <213> Hansenula polymorpha
<220>
   <223> Beschreibung der Sequenz: FMD-Promotorabschnitt
<400> 7
   atgagccagt 10
<210> 8
   <211> 10
   <212> DNA
   <213> Hansenula polymorpha
<220>
   <223> Beschreibung der Sequenz: FMD-Promotorabschnitt
<400> 8
   tccagatcat 10
<210> 9
   <211> 10.
   <212> DNA
   <213> Hansenula polymorpha
<220>
   <223> Beschreibung der Sequenz: FMD-Promotorabschnitt
<400> 9
   ggaggctcag 10
<210> 10
   <211> 10
   <212> DNA
   <213> Hansenula polymorpha
<220>
   <223> Beschreibung der Sequenz: FMD-Promotorabschnitt
<400> 10
   atttcgtaat 10
<210> 11
   <211> 10
<212> DNA
   <213> Hansenula polymorpha
<220>
   <223> Beschreibung der Sequenz: MOX-Promotorabschnitt
<400> 11
   atggtgtcat 10
<210> 12
   <211> 10
   <212> DNA
   <213> Hansenula polymorpha
<220>
   <223> Beschreibung der Sequenz: MOX-Promotorabschnitt
<400> 12
   ctttggtcat 10
<210> 13
   <211> 10
   <212> DNA
   <213> Hansenula polymorpha
<220>
   <223> Beschreibung der Sequenz: MOX-Promotorabschnitt
<400> 13
   cgaatgtaat 10
<210> 14
   <211> 10
   <212> DNA
   <213> Hansenula polymorpha
<220>
   <223> Beschreibung der Sequenz: MOX-Promotorabschnitt
<400> 14
   gaagcgtaat 10
<210> 15
   <211> 10
   <212> DNA
   <213> Hansenula polymorpha
<220>
   <223> Beschreibung der Sequenz: MOX-Promotorabschnitt
<400> 15
   gcccggtaat 10
<210> 16
   <211> 10
   <212> DNA
   <213> Hansenula polymorpha
<220>
   <223> Beschreibung der Sequenz: TPS1-Promotorabschnitt
<400> 16
   atggtgagat 10
<210> 17
   <211> 10
   <212> DNA
   <213> Hansenula polymorpha
<220>
   <223> Beschreibung der Sequenz: TPS1-Promotorabschnitt
<400> 17
   atgaaaggct 10
<210> 18
   <211> 10
   <212> DNA
   <213> Hansenula polymorpha
<220>
   <223> Beschreibung der Sequenz: TPS1-Promotorabschnitt
<400> 18
   atgaatctcc 10
<210> 19
   <211> 10
   <212> DNA
   <213> Hansenula polymorpha
<220>
   <223> Beschreibung der Sequenz: TPS1-Promotorabschnitt
<400> 19
   atgacgatat 10
<210> 20
   <211> 10
   <212> DNA
   <213> Hansenula polymorpha
<220>
   <223> Beschreibung der Sequenz: TPS1-Promotorabschnitt
<400> 20
   attgaccaat 10
<210> 21
   <211> 10
   <212> DNA
   <213> Hansenula polymorpha
<220>
   <223> Beschreibung der Sequenz: TPS1-Promotorabschnitt
<400> 21
   attgcaccca 10
<210> 22
   <211> 10
   <212> DNA
   <213> Hansenula polymorpha
<220>
   <223> Beschreibung der Sequenz: TPS1-Promotorabschnitt
<400> 22
   gattcgtaat 10
<210> 23
   <211> 10
   <212> DNA
   <213> Hansenula polymorpha
<220>
   <223> Beschreibung der Sequenz: TPS1-Promotorabschnitt
<400> 23
   ttttcgtcat 10
<210> 24
   <211> 1230
   <212> DNA
   <213> Hansenula polymorpha
<220>
   <223> Beschreibung der Sequenz: FMD-Promotor
<400> 24
   agcttgagca agttctcaaa cgcgggtttg tcgacgctct cccttatgcg actcctgcat      60
   taggaagcag cccagtagta ggttgaggcc gttgagcacc gccgccgcaa ggaatggtgt     120
   cgacgcggag aacgatctcc tcgacgcgga gaacgatctc ctcgagatcc aggaagcgca     180
   acgacgcggg gaatttcgta atcttccccg aggaaaaccg cttcccaaag atttcaccgg     240
   caataacgcc ttcattgacc gcacagagta ccacttgaac aacattctga aacggcagga     300
   ggcggttccg ccgtggatag agaaacaggg cagtgtggag gctcagatcc gccagtttcg     360
   gcgccagctc gactccgatt ggacactgca ggccgtgcac atcgtttgcg agaaacactc     420
   gaagaaaagc gacacggaca agatccagat catgagccag tacgccgagg cagagcgcaa     480
   caataactcg agttatacga agctgcgcag cagccaatgg gaacagaaat acagcaacta     540
   tctggaggtc aaattaaaag cgctgaatga cagcatccga ggttataatc tgcaggctcc     600
   tttggcatcg cagaaactgt atctaaacgc aaactccgag ctggaaaaat gttaccggcg     660
   atgcgcggac aatttagagg cggcgatcaa gaaacacctg ctgggcgagc agtctggagc     720
   acagtcttcg atgggcccga gatcccaccg cgttcctggg taccgggacg tgaggcagcg     780
   cgacatccat caaatatacc aggcgccaac cgagtctctc ggaaaacagc ttctggatat     840
   cttccgctgg cggcgcaacg acgaataata gtccctggag gtgacggaat atatatgtgt     900
   ggagggtaaa tctgacaggg tgtagcaaag gtaatatttt cctaaaacat gcaatcggct     960
   gccccgcaac gggaaaaaga atgactttgg cactcttcac cagagtgggg tgtcccgctc    1020
   gtgtgtgcaa ataggctccc actggtcacc ccggattttg cagaaaaaca gcaagttccg 1080
   gggtgtctca ctggtgtccg ccaataagag gagccggcag gcacggagtc tacatcaagc 1140
   tgtctccgat acactcgact accatccggg tctctcagag aggggaatgg cactataaat 1200
   accgcctcct gcgctctctg cttcatcggg 1230
<210> 25
   <211> 1508
   <212> DNA
   <213> Hansenula polymorpha
<220>
   <221> misc_feature
   <222> (1)..(1508)
   <223> n = a oder g oder c oder t/u, unbekannt oder sonstige
<220>
   <223> Beschreibung der Sequenz:MOX-Promotor
<400> 25
   agcttgcgga gaacgatctc ctcgagctgc tcgcggatca gcttgtggcc cggtaatgga 60
   accaggccga cgcgacgctc cttgcggacc acggtggctg gcgagcccag tttgtgaacg 120
   aggtcgttta gaacgtcctg cgcaaagtcc agtgtcagat gaatgtcctc ctcggaccaa 180
   ttcagcatgt tctcgagcag ccatctgtct ttggagtaga agcgtaatct ctgctcctcg 240
   ttactgtacc ggaagaggta gtttgcctcg ccgcccataa tgaacaggtt ctctttctgg 300
   tggcctgtga gcagcgggga cgtctggacg gcgtcgatga ggcccttgag gcgctcgtag 360
   tacttgttcc gtcgctgtag ccggccgcgg tgacgatacc cacatagagg tccttggcca 420
   ttagtttgat gaggtggggc aggatgggcg actcggcatc gaaatttttg ccgtcgtcgt 480
   acagtgtgat gtcaccatcg aatgtaatga gctgcagctt gcgatctcgg atggttttgg 540
   aatggaagaa ccgcgacatc tccaacagct gggccgtgtt gagaatgagc cggacgtcgt 600
   tgaacgaggg ggccacaagc cggcgtttgc tgatggcgcg gcgctcgtcc tcgatgtaga 660
   aggccttttc cagaggcagt ctcgtgaaga agctgccaac gctcggaacc agctgcacga 720
   gccgagacaa ttcgggggtg ccggctttgg tcatttcaat gttgtcgtcg atgaggagtt 780
   cgaggtcgtg gaagatttcc gcgtagcggc gttttgcctc agagtttacc atgaggtcgt 840
   ccactgcaga gatgccgttg ctcttcaccg cgtacaggac gaacggcgtg gccagcaggc 900
   ccttgatcca ttctatgagg ccatctcgac ggtgttcctt gagtgcgtac tccactctgt 960
   agcgactgga catctcgaga ctgggcttgc tgtgctggat gcaccaatta attgttgccg 1020
   catgcatcct tgcaccgcaa gtttttaaaa cccactcgct ttagccgtcg cgtaaaactt 1080
   gtgaatntgg caactgaggg ggttctgcag ccgcaaccga acttttcgct tcgaggacgc 1140
   agctggatgg tgtcatgtga ggctctgttt gctggggtag cctacaacgt gaccttgcct 1200
   aaccggacgg cgctacccac tgctgtctgt gcctgctacc agaaaatcac cagagcagca 1260
   gagggccgat gtggcaactg gtggggtgtc ggacaggctg tttctccaca gtgcaaatgc 1320
   gggtgaaccg gccagaaagt aaattcttat gctaccgtgc agcgactccg acatccccag 1380
   tttttgccct acttgatcac agatggggtc agcgctgccg ctaagtgtac ccaaccgtcc 1440
   ccacacggtc catctataaa tactgctgcc agtgcacggt ggtgacatca atctaaagta 1500
   caaaaacg 1508
<210> 26
   <211> 566
   <212> DNA
   <213> Hansenula polymorpha
<220>
   <223> Beschreibung der Sequenz: TPS1-Promotor
<400> 26
   gatgtcaaaa tgggggatca caaaagtaca ctcacgagga aaatcaaaac cttctcgtac     60
   ctttaacaca tacggaaatg atcgatcgat ttgagaagat tcctcaatga ttttcgtcat    120
   atataggtat ctgaggtatt tatggaccga ttcgtaataa catcatatac atcgcgcttt    180
   gtccctgtcc cagagatttc gatgaaaaaa gcgaatttta ttctaatatt tgaagcatgc    240
   caaacatggg gcagttgatt tgtgtgaggg taaaatatca tgaattgcac ccatcaaatg    300
   cagcaagata ttgaccaatc ctataataga aaacagactt accacaaata gattgtgatg    360
   acgatattat gaatctccag atgaaaggct cgaaagctat gaagcctctt gaaacttttc    420
   atggtgagat aatattttcg aaatttccac gaacttctaa aacgcaatta ttgaatataa    480
   aggaaaaata atatttccat atagcaagca aatcaagctg cactcctcat ccttaaaact    540
   aataaatctt acccatttga taccag                                         566